# EUROPEAN PATENT APPLICATION

(11) **EP 1 857 837 A1**
(43) Date of publication of application: **21.11.2007**
(21) Application number: 06712372.9
(22) Date of filing: 26.01.2006
(51) Int. Cl.: G01T 7/00, A61B 6/00, G01T 1/20, H02J 1/00, H02J 7/00

(54) **RADIATION IMAGE DETECTOR AND RADIATION IMAGING SYSTEM**

(30) Priority: 31.01.2005 JP 2005023724; 31.01.2005 JP 2005023744; 31.01.2005 JP 2005023884
(71) Applicant: Konica Minolta Medical & Graphic, Inc., Tokyo 191-8511 (JP)
(72) Inventor: OHARA, Hiromu, Tokyo, 1928505 (JP)
(74) Representative: Brown, George Laurence
(86) International application number: PCT/JP2006/301190
(87) International publication number: WO 2006/080377

(57) **Abstract**

A radiation image detector switchable between an imaging ready state which can detect radiation and an imaging standby state in which power consumption is less than that of the imaging ready state. The detector includes: a switching unit for giving an instruction of switching between the imaging ready state and the imaging standby state; a chargeable or replaceable battery provided as a power supply source for supplying power to a plurality of drive units; and a battery remaining power detecting section for detecting a remaining power of the battery. The radiation image detector includes a control unit for controlling running states of the plurality of drive units to switch between the imaging ready state and the imaging standby state according to the instruction from the switching unit and for controlling the battery remaining power detecting section. The control unit controls the battery remaining power detecting section to detect the remaining power of the battery when an imaging instruction for switching from the imaging ready state to the imaging standby state, is input from the switching unit.

## Description

### Technical Field

The present invention relates to a radiation image detector and a radiation imaging system, and more particularly to a radiation image detector and a radiation imaging system for imaging a radiation image as represented by an X-ray image.

### Background Art

In the field of medical diagnosis, there has been widely used a radiation image which is obtained by irradiating a subject with radiation such as X-ray and detecting an intensity distribution of the radiation having transmitted through the subject. In recent years, with respect to imaging, there has been proposed a radiation imaging system using an FPD (flat panel detector; radiation image detector), which detects the radiation, converts the detected radiation into electric energy, and detects the electric energy as radiation image data.

Recently, there has been developed a cassette-shaped FPD in which the FPD is accommodated in a cassette for the purpose of improving the portability and easy handling of the FPD (see, for example, Patent Document 1). Particularly, in order to utilize the portability of the FPD, there has been developed a cassette-shaped FPD which performs wireless communication with a console controlling the FPD. The wireless-type cassette FPD is not supplied power from other equipment, therefore has a battery embedded therein. In order to use the battery as long as possible, the cassette-shaped FPD is configured to switch between a state of large power consumption, e.g., at the time of radiographing(imaging ready state) and a state of small power consumption, e.g., at the time of standby (imaging standby state). In the imaging ready state, power is supplied to every part necessary for radiographing in the cassette-shaped FPD. On the other hand, in the imaging standby state, power is supplied to necessary parts s for at least receiving various instructions, that is, an electrode is not supplied to any part unnecessary for receiving various instructions although these parts are necessary for radiographing.
Patent Document 1: JP H6-342099A

### Disclosure of the Invention

### Problems to be solved by the Invention

Since the wireless-type cassette-shaped FPD is not supplied power from other equipment, sometimes power is not sufficiently supplied because of insufficient remaining power even if radiographing is performed after switching to the imaging ready state from the imaging standby state. When the power is not sufficiently supplied for radiographing, some malfunctions occur, for example, signals cannot be read, an accurate diagnosis cannot be performed because of an unclear radiation image even if the signals are read, and so on. Further, read image data are usually stored in a memory, but when the power is not sufficiently supplied, the memory does not work normally, and the image data are possible to be deleted. When the image data are to be transmitted to an external device such as a console, the transmission cannot be performed if the power supply is not enough. In any case mentioned above, a patient is forced to be radiographed again and unnecessarily exposed to radiation.

An object of the invention is to prevent radiographing from being performed under insufficient remaining power of a battery, to suppress frequency of re-radiographing, and hence to prevent a patient from unnecessary exposure to radiation.

### Means for Solving the Problem

The radiation image detector according to the invention of claim 1 is a radiation image detector switchable between an imaging ready state which can detect radiation and an imaging standby state in which power consumption is less than that of the imaging ready state, the detector comprising:
a switching unit for giving an instruction of switching between the imaging ready state and the imaging standby state;
a chargeable or replaceable battery provided as a power supply source for supplying power to a plurality of drive units;
a battery remaining power detecting section for detecting a remaining power of the battery; and
a control unit for controlling running states of the plurality of drive units to switch between the imaging ready state and the imaging standby state according to the instruction from the switching unit and for controlling the battery remaining power detecting section,
wherein the control unit controls the running states of the plurality of drive units to switch from the imaging standby state to the imaging ready state on the basis of a detected result of the remaining power of the battery, by the battery remaining power detecting section.

According to the invention of claim 1, the detector can be switched from the imaging standby state to the imaging ready state based on the detected result of the remaining power of the battery with the battery remaining power detecting section, and therefore when the remaining power of the battery is not sufficient, the detector can be controlled not to be switched to the imaging ready state. This allows prevention of radiographing under insufficient remaining power of the battery.

The invention of claim 2 is a radiation image detector of claim 1, wherein the control unit controls the running states of the plurality of drive units to switch between the imaging ready state and the imaging standby state on the basis of the detected result of the remaining power by the battery remaining power detecting section at the time when an radiographing instruction for switching from the imaging standby state to the imaging ready state is input from the switching unit.

According to the invention of claim 2, on the basis of the detected result of the remaining power detected by the battery remaining power detecting section at the time when the radiographing instruction is input from the switching unit, the detector can be switched between the imaging ready state and the imaging standby state, thereby being automatically switched to a state suitable for the remaining power of the battery prior to radiographing.

The invention of claim 3 is a radiation image detector of claim 2, wherein the control unit controls the running states of the plurality of drive units such that the detector goes into the imaging ready state when the detected result of the remaining power in the battery remaining power detecting section, at the time when the radiographing instruction is input from the switching unit, satisfies the power possible to radiographto radiograph, and goes into the imaging standby state when the detected result is less than the power possible to radiographto radiograph.

According to the invention of claim 3, the detector goes into the imaging ready state when the detected result of the remaining power in the battery remaining power detecting section, at the time when the radiographing instruction is input from the switching unit, satisfies the power possible to radiographto radiograph, and goes into the imaging standby state when the detected result is less than the power possible to radiographto radiograph. This can securely prevent the radiographing from being performed with the remaining power less than that possible to radiographto radiograph.

The invention of claim 4 is a radiation image detector of claim 3, further comprising a notifying unit for notifying on the basis of the control of the control unit, wherein the control unit controls the notifying unit to notify that the radiographing is not permitted when the detected result of the remaining power in the battery remaining power detecting section, at the time when the radiographing instruction is input from the switching unit, is less than the power possible to radiographto radiograph.

According to the invention of claim 4, at the time when the radiographing instruction is input from the switching unit, the notifying unit notifies that the radiographing is not permitted when the detected result of the remaining power in the battery remaining power detecting section, is less than the power possible to radiographto radiograph, and therefore the radiographer can carry out, e.g., replacement, charging of the battery based on the notice.

The invention of claim 5 is a radiation image detector of any one of claims 1 to 4, wherein the imaging standby state has a plurality of modes, and wherein the control unit controls the running states of the plurality of drive units so that the plurality of modes have respective different power consumptions.

According to the invention of claim 5, the imaging standby state includes a plurality of modes with respective different power consumptions, and therefore the radiation image detector can be set to the most suitable state according to, e.g., its use condition after completion of charging or replacement of the battery. This allows suppression of useless power consumption, and allows efficient radiographing work.

The invention of claim 6 is a radiation image detector of claim 5, wherein the control unit controls the running states of the plurality of drive units such that the detector goes into a mode of minimum power consumption in the plurality of modes in the imaging standby state when the detected result of the remaining power in the battery remaining power detecting section, at the time when the radiographing instruction is input from the switching unit, is less than the power possible to radiographto radiograph.

According to the invention of claim 6, when the detected result of the remaining power in the battery remaining power detecting section, at the time when the radiographing instruction is input from the switching unit, is less than the power possible to radiographto radiograph, the detector goes into the mode of minimum power consumption out of the plurality of modes in the imaging standby state, and therefore the power consumption can be reduced as much as possible when radiographing is not permitted.

The invention of claim 7 is a radiation image detector of claims 5 or 6, wherein the control unit controls the battery remaining power detecting section to detect the remaining power of the battery when a standby-state switching instruction for switching from a mode of smaller power consumption to a mode of larger power consumption in the plurality of modes is input from the switching unit, the standby-state switching instruction.

When provided with a plurality of imaging standby states with respective different power consumptions, it is possible to switch the imaging standby state according to a condition at the time of standby. The radiation image detector includes parts which have a characteristic of deterioration with time when power is supplied (such as photodiodes and thin film transistors). The photodiodes and the thin film transistors need a longer time to go into their stable states when power is supplied again after stopped. Accordingly, it is possible that: when radiographing is not performed for a while, an imaging standby mode, in which power is not supplied to the photodiodes and the thin film transistors, is set, and when radiographing will soon be performed, an imaging standby mode, in which power is supplied to the photodiodes and the thin film transistors, is set. On the contrary, ICs for reading and the like have large power consumption and need not a longer time to go into their stable states, therefore may be set to an imaging standby mode in which power is not supplied until just before radiographing. As will be understood from these examples, when the imaging standby state of less power consumption is transferred to the imaging standby state of larger power consumption out of the plurality of imaging standby states, it is probable that radiographing will soon be performed. That is, according to the invention as described in claim 7, if the remaining power of the battery is detected when a standby-state switching instruction, for switching from the imaging standby state of less power consumption to the imaging standby state of larger power consumption, is input from the switching unit, the remaining power of the battery can be recognized prior to radiographing. This allows determination prior to the radiographing as to whether the detector can perform radiographing normally, and hence prevents the radiographing from being performed under insufficient remaining power of the battery.

The invention of claim 8 is a radiation image detector of any one of claims 1 to 7, further comprising:
a check unit for performing an operation check of the drive units as to whether the drive units can work normally when the detector starts to operate; and
a control unit for controlling the running states of the plurality of drive units on the basis of the result of the operation checks.

According to the invention of claim 8, the radiation image detector, which detects irradiated radiation to obtain radiation image data, includes the plurality of drive units, the check unit for performing operation checks of the drive units as to whether the drive units can work normally when the detector starts operating, and a control unit for controlling the running states of the plurality of drive units based on the result of the operation checks. Therefore, the radiation image detector can perform operation checks of the drive units prior to radiographing. This allows determination prior to the radiographing as to whether the drive units can work normally, and hence prevents the radiographing from being performed while the drive units do not work normally.

The invention of claim 9 is a radiation image detector of claim 8,
wherein the running state includes an ON state of a main power source, the ON state including an imaging ready state which can detect radiation and an imaging standby state in which power consumption is less than that of the imaging ready state; and an OFF state of the main power source in which power supply to the drive units is completely shut off, and
wherein when the check unit detects that one of the drive units cannot work normally, the detector is not switched at least to the imaging ready state.

According to the invention of claim 9, the running state includes an ON state of a main power source, the ON state including an imaging ready state which can detect radiation and an imaging standby state in which power consumption is less than that in the imaging ready state; and an OFF state of the main power source in which power supply to the drive units is completely shut off. When the check unit detects that some of the drive units cannot work normally, the detector is not switched at least to the imaging ready state. Accordingly, the running state of the radiation image detector can be switched between the ON state, including the imaging ready state and the imaging standby state, and the OFF state of the main power source, and further the control unit controls the plurality of drive units so as to switch to the imaging standby state or the OFF state of the main power source when the check unit detects as the result of the operation checks that respective drive units cannot work normally.

The invention of claim 10 is a radiation image detector of claim 9, wherein when the battery remaining power detecting section detects that the remaining power of the power source is less than a predetermined power possible to radiographto radiograph, the control unit causes the detector to go into the OFF state of the main power source.

According to the invention of claim 10, when the battery remaining power detecting section detects that the remaining power of the power source is less than a predetermined power possible to radiographradiograph, the control unit causes the detector to go into the OFF state of the main power source. Therefore, in the radiation image detector, when the result of the remaining power, detected by the battery remaining power detecting section, indicates that the remaining power of the power source is less than a predetermined power possible to radiograph, the control unit controls the running states of the plurality of drive units so that the detector is in the OFF state of the main power source, and prevents the detector from starting operation.

The invention of claim 11 is a radiation image detector of claim 9,
wherein the imaging standby state includes a first imaging standby mode, and a second imaging standby mode in which power consumption is less than that of the first imaging standby mode, and
the detector further comprises a communication unit as the drive unit; and a communication check unit for performing communication check of the communication unit as the operation check.

According to the invention of claim 11, the imaging standby state includes the first imaging standby mode, and the second imaging standby mode in which power consumption is less than that in the first imaging standby mode. The detector further includes the communication unit as the drive unit, and the communication check unit for performing a communication check of the communication unit as the operation check. Therefore, the control unit can control the running states of the plurality of drive units according to the result of the communication check.

The invention of claim 12 is a radiation image detector of claim 11, wherein when the communication check unit detects that the communication unit cannot work normally, the control unit causes the detector to go into the second imaging standby mode.

According to the invention of claim 12, when the communication check unit detects that the communication unit cannot work normally, the control unit causes the detector to go into the second imaging standby mode. Therefore, the control unit controls the running states of the plurality of drive units so that the detector can go into the second imaging standby mode when the communication check unit detects that the communication unit cannot work normally, and enables the radiation image detector to run in a state of less power consumption.

The invention of claim 13 is a radiation image detector of claim 14, further comprising a notifying unit for performing notification on the basis of the control of the control unit, wherein when the communication check unit detects that the communication unit cannot work normally, the notifying unit notifies that the communication unit is impossible to work normally.

According to the invention of claim 13, the detector further includes a notifying unit for notifying based on the control of the control unit, and when the communication check unit detects that the communication unit cannot work normally, the notifying unit notifies that the communication unit is impossible to work normally. Therefore, the control unit can notify through the notifying unit that the communication unit is impossible to work normally when the communication unit cannot work normally.

The invention of claim 14 is a radiation image detector of claim 9,
wherein the imaging standby state includes a first imaging standby mode, and a second imaging standby mode in which power consumption is less than that of the first imaging standby mode, and
the detector further comprises an image storing unit as the drive unit; and a memory check unit for performing a memory check of the image storing unit as the operation check.

According to the invention of claim 14, the imaging standby state includes the first imaging standby mode, and the second imaging standby mode in which power consumption is less than that in the first imaging standby mode. The detector further includes an image storing unit as the drive unit, and a memory check unit for performing a memory check of the image storing unit as the operation check. Therefore, the control unit can control the running states of the plurality of drive units according to the result of the memory check of the image storing unit. the control unit controls the running states of the plurality of drive units so that the detector can go into the second imaging standby mode when, and enables the radiation image detector to run in a state of less power consumption.

The invention of claim 15 is a radiation image detector of claim 14, wherein when the memory check unit detects that the image storing unit cannot work normally, the control unit causes the detector to go into the second imaging standby mode.

According to the invention of claim 15, when the memory check unit detects that the image storing unit cannot work normally, the control unit causes the detector to go into the second imaging standby mode. Therefore, the control unit controls the running states of the plurality of drive units so that the detector can go into the second imaging standby mode when the memory check unit detects that the image storing unit cannot work normally, and enables the radiation image detector to run in a state of less power consumption.

The invention of claim 16 is a radiation image detector of any one of claims 1 to 15, wherein the detector is a cassette-shaped flat panel detector which detects irradiated radiation, converts the radiation to electric signals, accumulates the electric signals, and reads the accumulated electric signals to obtain radiation image data.

According to the invention of claim 16, the radiation image detector is a cassette-shaped FPD, therefore possible to be easily carried regardless of radiographing places, thereby improving flexibility of radiographing. Moreover, even when such a radiation image detector is used for radiographing, the radiation image detector can be set to the imaging ready state or the imaging standby state according to, e.g., its use condition after completion of charging or replacement of the battery, whereby the invention achieves effects that useless power consumption is suppressed and efficient radiographing work can be performed.

The invention of claim 17 is a radiation imaging system comprising:
the radiation image detector of any one of claims 1 to 16; and
a console which controls the radiation image detector.

According to the invention of claim 17, the radiation imaging system can achieve the same actions and effects as of the inventions described in claims 1 to 16.

The invention of claim 18 is a radiation imaging system of claim 17,
wherein the console comprises a display unit which displays on the basis of the control of the control unit, and
the control unit controls the display unit to display that the radiographing is not permitted when the detected result of the remaining power in the battery remaining power detecting section, at the time when the radiographing instruction is input from the switching unit, is less than the power possible to radiographto radiograph.

According to the invention of claim 18, the invention can achieve the same action and effect as of the invention of claim 4.

The invention of claim 19 is a radiation imaging system of claim 18, wherein the control unit controls the display unit to display the remaining power of the battery on the basis of the detected result in the battery remaining power detecting section.

According to the invention of claim 19, the remaining power of the battery is displayed on the display unit of the console, whereby the remaining power of the battery can be visually recognized. This allows speedy dealing of charging or replacement of the battery.

The invention of claim 20 is a radiation imaging system of claims 18 or 19, wherein the control unit controls the display unit to display whether the radiation image detector is in the imaging ready state or the imaging standby state.

According to the invention of claim 20, there is displayed on the display unit of the console as to whether the radiation image detector is in the imaging ready state or the imaging standby state. Therefore, the present state of the radiation image detector can be visually recognized.

The invention of claim 21 is a radiation imaging system comprising:
the radiation image detector of any one of claims 8 to 15; and
a console which controls the radiation image detector,
wherein the console comprises a notifying unit which notifies either one or both of the running state of the radiation image detector and the result of operation checks of the radiation image detector.

According to the invention of claim 21, the console includes a notifying unit which notifies either one or both of the running state of the radiation image detector and the result of operation checks of the radiation image detector. Therefore, the console can notify, through the notifying unit, the running state and checked state by the check unit in the radiation image detector.

### Effects of the Invention

According to the invention, it is possible to determine prior to radiographing whether normal radiographing can be performed. Therefore, radiographing with insufficient remaining power of a battery can be prevented, the frequency of re-radiographing is suppressed, and a patient can be prevented from unnecessary exposure to radiation.

### Brief Description of the Drawings

[FIG. 1] This is a view showing a schematic configuration illustrating one embodiment of a radiation imaging system according to the present invention.
[FIG. 2] This is a perspective view showing a structure of main elements of a radiation image detector according to the present invention.
[FIG. 3] This is a block diagram showing a configuration of main units of the radiation image detector according to the present invention.
[FIG. 4] This is an equivalent circuit diagram for one pixel in a photoelectric conversion unit constituting a signal detection unit included in the radiation image detector of FIG. 2.
[FIG. 5] This is an equivalent circuit diagram in which the photoelectric conversion units shown in FIG. 4 are arranged two-dimensionally.
[FIG. 6] This is a block diagram showing a configuration of main units of a console included in the radiation imaging system of FIG. 1.
[FIG. 7] This is a perspective view showing a radiation image detector of a third embodiment.
[FIG. 8] This is a block diagram showing a configuration of main units of the radiation image detector of the third embodiment.

### Preferred embodiments of the Invention

### [First Embodiment]

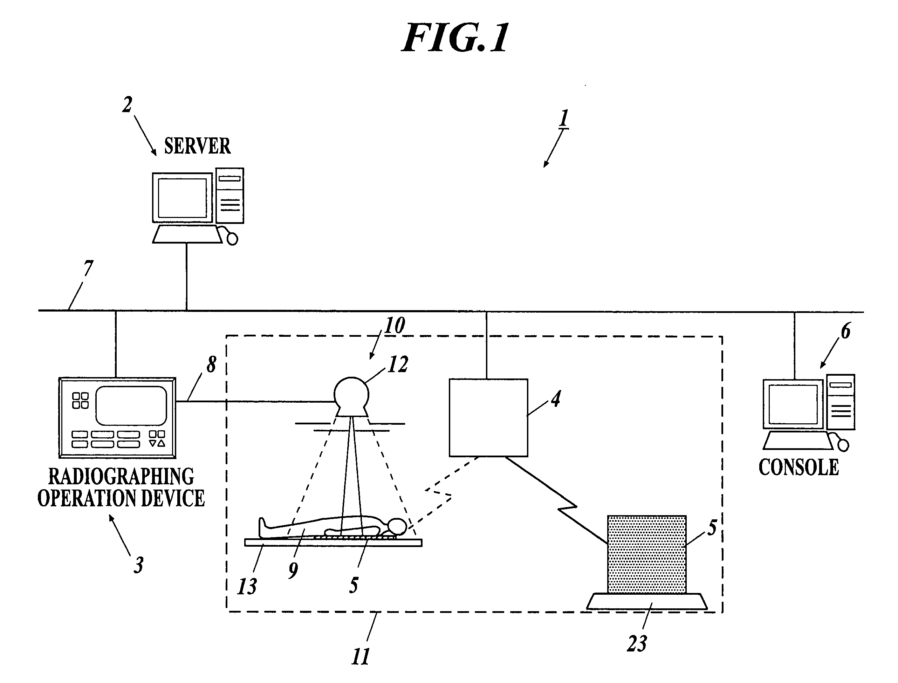

An embodiment of the present invention will be described below with reference to FIGS. 1 to 6.

FIG. 1 is a view showing a schematic configuration of one embodiment of a radiation imaging system including a radiation image detector according to the invention applied thereto.

A radiation imaging system 1 of the embodiment is a system, which is, for example, applied to radiation imaging performed in a hospital. As shown in FIG. 1, the system 1 includes a server 2 that manages various kinds of information concerning the radiographing and a patient, an radiographing operation device 3 that performs operations associated with the radiation imaging, a base station 4 that performs communication, e.g., by a wireless communication system such as a wireless LAN (local area network), a console 6 that controls a radiation image detector 5 and executes image processing on a radiation image detected by the radiation image detector 5, and a network 7 through which devices 2, 3, 4, 5 and 6 are connected to each other. The radiographing operation device 3 is connected through a cable 8 to a radiation imaging device 10 that irradiates the patient as a subject 9 with radiation to perform radiation imaging. The radiation imaging device 10 and radiation image detector 5 are, for example, installed by one device in one radiographing room 11, and radiation image data can be obtained by operating the radiation imaging device 10 with the radiographing operation device 3 and detecting the radiation image with the radiation image detector 5. Alternatively, a plurality of radiation image detectors 5 may be provided in one radiographing room 11.

The network 7 may be a communication line dedicated to the system; however, the network 7 is preferably an existing line such as Ethernet (registered trademark) because otherwise the flexibility of system configuration would be reduced, or for other reasons. In addition to the devices represented above, there may be connected to the network 7 a plurality of radiographing operation devices 3 that operate radiation imaging devices 10 installed in other radiographing rooms 11, radiation image detectors 5, and consoles 6.

First, the radiographing operation device 3 includes an input operation unit(not shown) that operates the radiation imaging device 10 by, e.g., inputting signals of radiographing conditions or the like, the input operation unit(not shown) including an operation panel and the like; a display unit(not shown) that displays information about the radiographing conditions etc., various instructions and the like; and a power supply unit(not shown) that supplies power to the radiation imaging device 10.

The radiation imaging device 10 is arranged in the radiographing room 11. The radiation imaging device 10 includes a radiation source 12. radiation is generated by applying a tube voltage to the radiation source 12. for example, a radiation tube is used for the radiation source 12. The radiation tube generates radiation by colliding accelerated electrons generated by thermal excitation with a cathode under a high voltage.

The radiation image detector 5 detects radiation emitted from the radiation source 12 of the radiation radiographing device 10 and transmitted through the subject 9, and acquires a radiation image. The radiation image detector 5 is disposed within the coverage of the radiation emitted from the radiation source 12 when radiographing is performed. The radiographic image detector 5 is disposed, for example, as shown in FIG. 1, between the subject 9 and a bed 13 on which the subject is laid. However, the position thereof is not limited thereto. For example, there may be provided below the bed a detector mounting opening (not shown) through which the radiation image detector 5 is to be mounted, and the radiation image detector 5 may be inserted into the detector mounting opening.

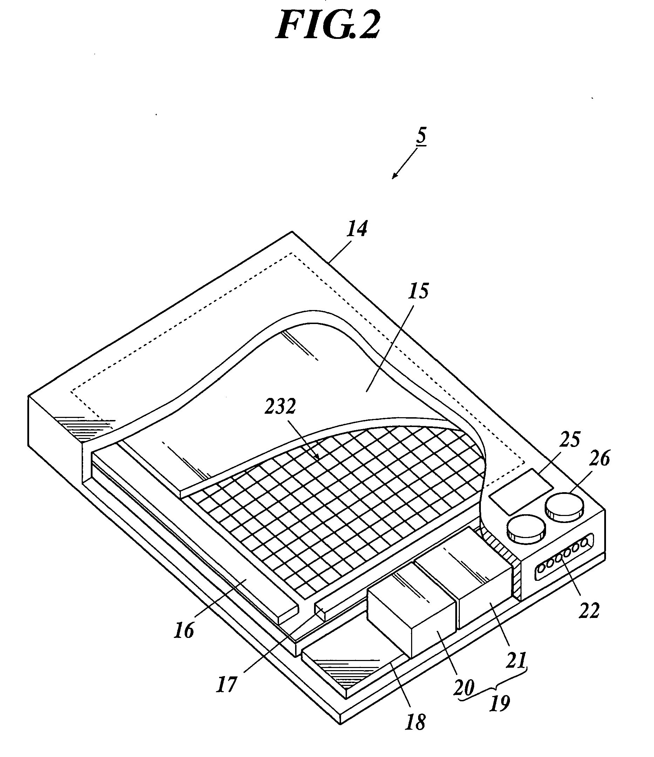

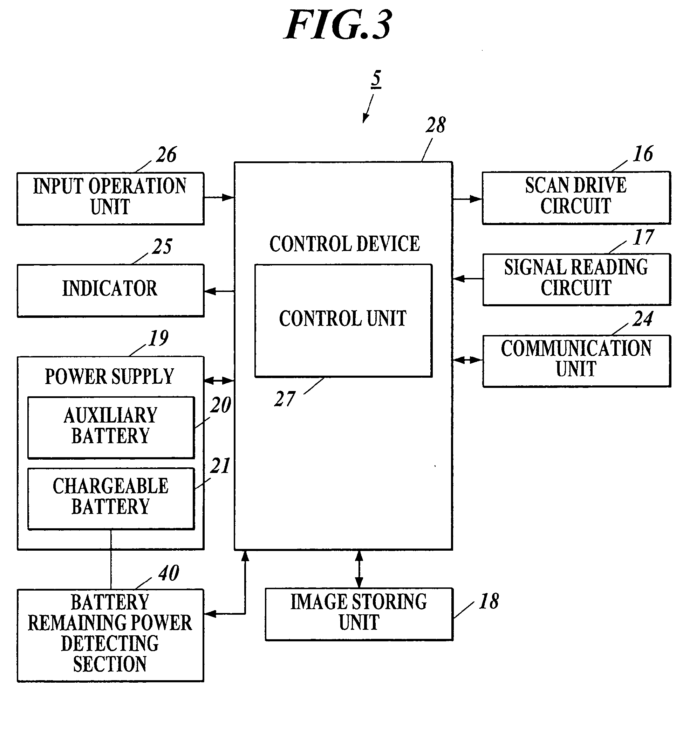

The radiation image detector 5 is a cassette-shaped flat panel detector. A structure of the radiation image detector 5 will be described below with reference to FIGS. 2 and 3.

As shown in FIG. 2, the radiation image detector 5 includes a casing 14 to protect the inside of the detector, and is configured to be portable as a cassette.

Inside the casing 14, there is formed a layered imaging panel 15 to convert irradiated radiation into an electric signal. On a surface-to-be-irradiated of the imaging panel 15, there is provided a light-emitting layer (not shown) to emit light in accordance with intensity of the radiation which is incident thereon.

The light-emitting layer is one generally called a scintillator layer, and, for example, contains phosphor as a main component and outputs an electromagnetic wave with a wavelength of 300 to 800 nm according to the incident radiation, namely, an electromagnetic wave (light) ranging from ultraviolet light to infrared light including visible light in the midst.

For the phosphor to be used in the light-emitting layer, for example, phosphor containing CaWO.sub.4 or the like as a basic substance and phosphor formed by actively imparting a main light-emitting substance into a basic substance such as CsI:Tl, Gd.sub.20.sub.2S:Tb, and ZnS:Ag may be used. Moreover, phosphor represented by a general formula (Gd, M, Eu).sub.20.sub.3 where M is a rare-earth element can be used. Particularly, CsI:Tl and Gd.sub.20.sub.2S:Tb are preferable because of high radiation absorption and light-emitting efficiencies thereof. By using these substances, a low-noise and high-quality image can be obtained.

On the surface opposite to the surface-to-be-irradiated of the light-emitting layer, there is formed a signal detection unit 232 which converts the electromagnetic wave (light) output from the light-emitting layer into electric energy and accumulates the electric energy therein. The signal detection unit 232 further outputs an image signal based on the accumulated electric energy.

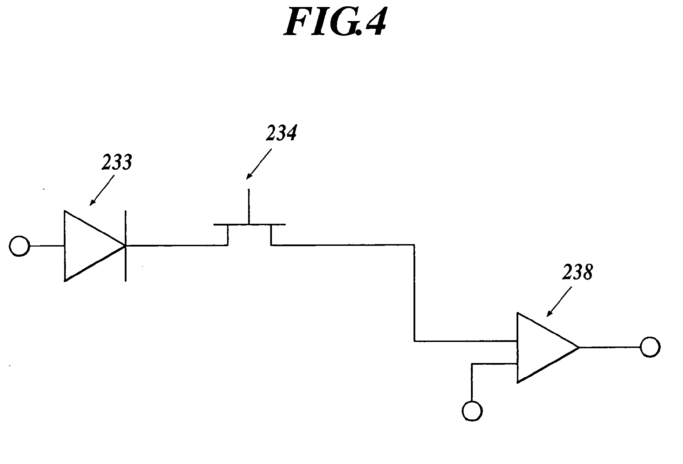

A circuit configuration of the imaging panel 15 will now be described. FIG. 4 is an equivalent circuit diagram for one pixel of a photoelectric conversion unit constituting the signal detection unit 232.

As shown in FIG. 4, one pixel of the photoelectric conversion unit includes a photodiode 233, and a thin-film transistor (hereinafter, "TFT") 234 that extracts electric energy accumulated in the photodiode 233 as an electric signal by switching. The extracted electric signal is amplified by an amplifier 238 to such a level that a signal reading circuit 237 can detect the electric signal. A reset circuit (not shown) including a TFT 234 and a capacitor is connected to the amplifier 238. The reset circuit performs a reset operation for resetting the accumulated electric signal by switching on the TFT 234. The photodiode 233 may be a photodiode simply having a parasitic capacitance, or may include an additional capacitor in parallel in order to improve dynamic ranges of the photodiode 233 and the photoelectric conversion unit.

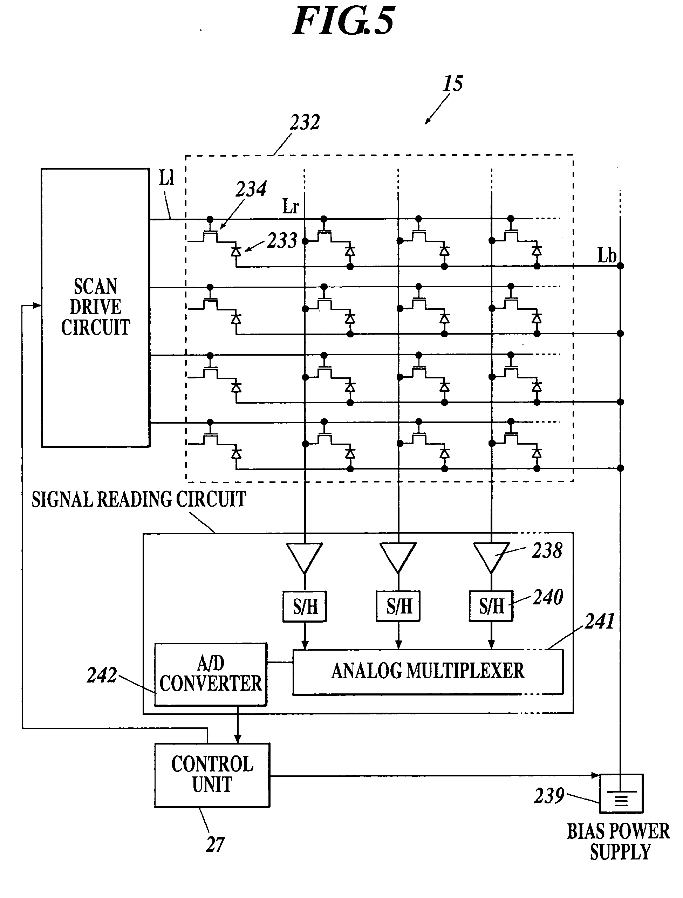

FIG. 5 is an equivalent circuit diagram in which the above-described photoelectric conversion units are arranged two-dimensionally. Between the pixels, scan lines Ll and signal lines Lr are arranged to be perpendicular to each other. A TFT 234 is connected to each photodiode 233 described above, and one end of the photodiode 233 on a side to which the TFT 234 is connected is connected to the signal line Lr. The other end of the photodiode 233 is connected to one end of the adjacent photodiode 233 arranged on each row, and connected to a bias power supply 239 through a common bias line Lb. One end of the bias power supply 239 is connected to a control unit 27, and thus a voltage is applied to the photodiodes 233 through the bias line Lb according to an instruction from the control unit 27. The TFTs 234, arranged on each row, are connected to their common scan line L1, and each scan line L1 is connected to the control unit 27 through a scan drive circuit 236. Similarly, the photodiodes 233 arranged on each column are connected to their common signal line Lr, and connected to the signal reading circuit 237 controlled by the control unit 27. In the signal reading circuit 237, an amplifier 238, a sample/hold circuit 240, an analog multiplexer 241 and an A/D converter 242 are arranged on the common signal line Lr in this order when viewed from the imaging panel 15.
The TFT 234 may be of an inorganic semiconductor series or one using an organic semiconductor, which is used in a liquid crystal display and the like.
Although the photodiodes 233 are used as the photoelectric conversion elements in this embodiment, solid-state imaging elements other than the photodiodes may be used as the photoelectric conversion elements.

As shown in FIG. 2, on side portions of the signal detection unit 232, there are disposed the scan drive circuit 16 to scan and drive the respective photoelectric conversion elements by sending pulses to the photoelectric conversion elements, and the signal reading circuit 17 to read the electric energy accumulated in the respective photoelectric conversion elements.

As shown in FIGS. 2 and 3, the radiation image detector 5 includes an image storing unit 18 which is, for example, a rewritable memory such as a RAM (random access memory) or a flash memory. The image storing unit 18 stores image signals output from the imaging panel 15. The image storing unit 18 may be a built-in memory or a detachable memory such as a memory card.

The radiation image detector 5 is provided with a power supply 19 as a power supply source for supplying power to a plurality of drive units (e.g., scan drive circuit 16, signal reading circuit 17, communication unit 24 (described later), image storing unit 18, battery remaining power detecting section 40 (described later), indicator 25 (described later), input operation unit 26 (described later), imaging panel 15, etc.) constituting the radiation image detector 5. The power supply 19 includes an auxiliary battery 20 and a chargeable battery 21. The auxiliary battery 20 includes, e.g., manganese battery, alkaline battery, alkaline button battery, lithium battery, silver oxide battery, air-zinc battery, nickel-cadmium battery, mercury battery and lead battery. The chargeable battery 21 includes, e.g., nickel-cadmium battery, nickel-hydrogen battery, lithium-ion battery, small sealed lead battery, lead-acid battery, fuel cell, and solar cell. By providing the auxiliary battery 20 other than the chargeable battery 21, it becomes possible to supply power to the radiation image detector 5 at least at minimum power when the charged amount of the battery 21 is insufficient or during replacement of the battery 21. This auxiliary function prevents the detector 5 from erroneously deleting the image data stored in the image storage unit 18, and from getting unable to receive a signal from an external device such as the console 6.

On one end of the casing 14, there are provided a connection terminal 22 for charging. For example, as shown in FIG. 1, by attaching the radiation image detector 5 onto a charging device 23 such as a cradle, the terminal 22 of the casing is coupled to a terminal (not shown) on the charging device 23, and the chargeable battery 21 is charged. The chargeable battery 21 is mounted removable from the side of the casing 14 for replacement. The shape of the auxiliary battery 20 and the chargeable battery 21, included in the power supply 19, are not limited to that illustrated in FIG. 2. For example, a battery formed in a plate shape may be provided in parallel to the imaging panel 15. By forming each battery into such a shape, a ratio of the imaging panel surface to the casing 14 is increased and thus an effective imaging area can be increased. With this shape, whole size of the radiation image detector 5 can be made smaller relative to the same imaging area, and resultantly, the radiation image detector 5 can be made thinner.

Further, the radiation image detector 5 is provided with a communication unit 24 (see FIG. 3) for transmitting and receiving various signals to and from an external device such as the console 6. The communication unit 24, for example, transmits an image signal output from the imaging panel 15 to the console 6, and receives an radiographing instruction signal, a standby instruction signal, etc. sent from the console 6 or the like.

Moreover, at one end on the surface of the casing 14, an indicator (notifying unit) 25 is provided for displaying and notifying the charging state of the chargeable battery 21, various operation states and the like, so that an operator can visually confirm the charging state of the chargeable battery 21 and the like of the radiation image detector 5.

On the outer side of the casing 14, there is provided the input operation unit 26 for inputting the radiographing instruction and the standby instruction. The radiation image detector 5 has as operation states an imaging ready state and an imaging standby state in which the power consumption is less than that of the imaging ready state, and these states can be switched with the input operation unit 26 operated. For example, when the radiographing instruction is input to the input operation unit 26 or when an radiographing instruction signal is input from the console 6 to the communication unit 24, the imaging ready state is set. On the other hand, when the standby instruction is input to the input operation unit 26 or when a standby instruction signal is input from the console 6 to the communication unit 24, the imaging standby state is set. Thus, the input operation unit 26 and the communication unit 24 serve as a switching unit for switching the imaging ready state and the imaging standby state according to the invention.

Hereinafter, the imaging ready state and the imaging standby state will be described.
The imaging ready state is a state in which all units, included in the radiation image detector 5 and used in a series of radiographing operations, work, that is, power is supplied to all units used in the series of radiographing operations, such as scan drive circuit 16, signal reading circuit 17, photodiodes 233, TFTs 234, image storing unit 18, and communication unit 24. In this state, it is possible to perform respective operations of the series of radiographing operations, such as initialization of image data, accumulation of electric energy generated according to the irradiated radiation, reading of electric signals, and transmission of image signals. In the initialization, the reset operation and a offset image reading operation in the imaging panel 15 are performed. The series of radiographing operations mean respective operations such as initialization of image data, accumulation of electric energy generated depending on the irradiated radiation, reading of electric signals, and transmission of image signals.

In this embodiment, the imaging standby state includes a first imaging standby mode in which power consumption is less than that of the imaging ready state, and a second imaging standby mode in which power consumption is less than that of the first imaging standby mode.

The first imaging standby mode is the imaging standby state in which all units used in the series of radiographing operations are active except the signal reading circuit 17 so as to go into the imaging ready state rapidly, and which is ready to perform radiographing. Specifically, it is the state in which power is supplied to respective units such as scan drive circuit 16, photodiodes 233, TFTs 234, image storing unit 18, and communication unit 24. The second imaging standby mode is the imaging standby state in which only the image storing unit 18, associated with storing of images, and the communication unit 24, associated with transmission of image data to the outside and reception of signals from the outside, are active, and which is not ready to perform radiographing and in a state of very low power consumption.

As shown in FIG. 3, the radiation image detector 5 includes a control device 28 provided with the control unit (hereinafter, simply "controller") 27 having, for example, a general-purpose CPU, ROM, RAM and the like (none of them are shown). The controller 27 reads out a predetermined program stored in the ROM to develop the program in a work area of the RAM, and enables the CPU to execute various kinds of processing according to the program.
The ROM stores various kinds of control data in addition to the programs. The control data include, for example, remaining power determining data for determining whether the remaining power of the chargeable battery 21 satisfies the power possible to radiographto radiograph.

The radiation image detector 5 further includes a battery remaining power detecting section 40 for detecting the remaining power of the chargeable battery 21. The battery remaining power detecting section 40 detects the remaining power of the chargeable battery 21 with control of the controller 27, and outputs the obtained battery remaining power to the controller 27. It is possible to employ various timings for detecting the battery remaining power, and in this embodiment, the control unit 27 controls the battery remaining power detecting unit 40 so as to detect the remaining power of the chargeable battery 21 at least when an instruction of switching from the imaging standby state to the imaging ready state (radiographing instruction) is input from the input operation unit 26 or the communication unit 24.

Based on the detected result of the remaining power at the time when the imaging instruction is input from the input operation unit 26 or the communication unit 24, the controller 27 switches between the imaging ready state and the imaging standby state. Specifically, the controller 27 compares the detected result of the remaining power at the time when the radiographing instruction is input with the remaining power determining data, and controls respective running states of the plurality of drive units to switch to the imaging ready state when the detected result of the remaining power satisfies the power possible to radiographto radiograph. On the other hand, when the detected result is less than the power possible to radiographto radiograph, the controller 27 controls respective running states of the plurality of drive units so that the detector goes into the mode of minimum power consumption, namely, the second imaging standby mode. The drive control of respective units results in control of the power consumption of the battery.

When the detected result of the remaining power is input to the controller 27 from the battery remaining power detecting section 40, the controller indicates the remaining power of the chargeable battery 21 on the indicator 25 based on the detected result. At this time, when the detected result of the remaining power is less than the power possible to radiographto radiograph, the controller 27 controls the indicator 25 to display that radiographing is not permitted. The controller 27 further transmits a signal indicating the state to the console 6 through the communication unit 24.

The information input from the input operation unit 26 and the signal received from the communication unit 24 are sent to the controller 27, and the controller 27 controls the respective drive units based on the sent signals.

The controller unit 27 drives the scan drive circuit 16 to send the pulse signals to the respective photoelectric conversion elements, thus scanning and driving the respective photoelectric conversion elements. Then, the image signal is read by the signal reading circuit 17 which reads the electric energy accumulated in the respective photoelectric conversion elements, and the image signal thus read is sent to the controller 27. The controller 27 enables the image storing unit 18 to store the sent image signal. The image signal stored in the image storing unit 18 is sent through the communication unit 24 to the console 6 as appropriate.

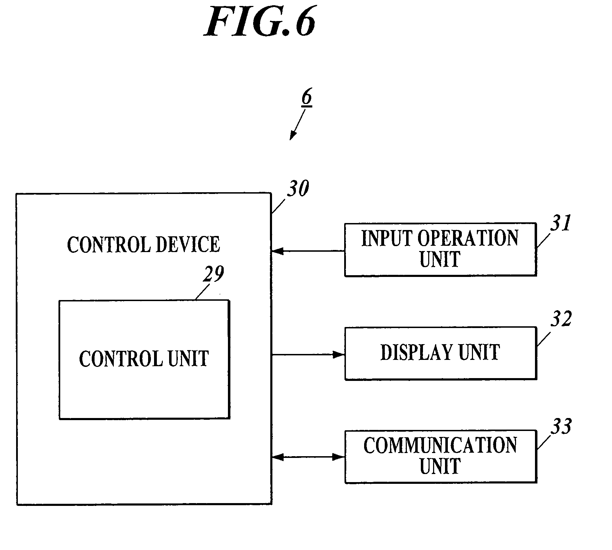

As shown in FIG. 6, the console 6 includes a control device 30 including a control unit 29 which includes, for example, a general-purpose CPU, ROM, RAM and the like (none of them are shown). The control unit 29 reads predetermined programs stored in the ROM to develop the programs in a work area of the RAM, and enables the CPU to execute various kinds of processing according to the programs.

Moreover, the console 6 includes an input operation unit 31 that inputs various types of instructions and the like, a display unit 32 that displays an image, various messages and the like, and a communication unit 33 that transmits and receives a signal to and from an external device such as the radiation image detector 5.

The input operation unit 31 includes, for example, an operation panel, a keyboard, a mouse and the like, and outputs a depression signal sent from a depressed key on the operation panel or keyboard and an operation signal sent from the mouse, to the control unit 29 as an input signal.

The display unit 32 includes, for example, a CRT (cathode ray tube), an LCD (liquid crystal display) and the like, and displays various screens according to an instruction of a display signal output from the control unit 29.

The communication unit 33 communicates various types of information with the radiation image detector 5 through the base station 4 using a wireless communication system such as a wireless LAN.

A signal input from the input operation unit 31, a signal received from the outside through the communication unit 33 and the like are sent to the control unit 29, which executes predetermined processing on the sent signals. For example, the radiation image data detected by the radiation image detector 5 is converted into signals and sent to the control unit 29. The control unit 29 executes the predetermined image processing based on the signals, to thereby obtain a radiation image. Further, the control unit 29 enables the display unit 32 to display the radiation image, a thumbnail image, various types of information input from the input unit, the remaining power of the chargeable battery 21 based on the detected result from the battery remaining power detecting section 40, the state of the radiation image detector 5 (the imaging ready state or the imaging standby state), and the like.

A description will now be given of an action of the radiation imaging system 1 including the radiation image detector 5 applied thereto according to the embodiment.

When a radiographing-reservation is not input to the radiation image detector 5, the controller 27 of the radiation image detector 5 normally controls respective running states of the plurality of drive units for the first imaging standby mode so as to start radiographing upon receiving a reservation.

Thereafter, when an radiographing-reservation instruction is input to the console 6, a radiographer selects a radiation image detector 5 to be used for the radiographing on the console 6, and inputs the choice of detector to the input operation unit 31 of the console 6. The input choice of detector is transmitted to the communication unit 24 of the selected detector 5 through the communication unit 33 of the console 6, and input to the controller 27, for example, as the radiographing instruction information. Based on the radiographing instruction information, the controller 27 controls the power consumption of the chargeable battery 21 for switching from the first radiographing standby mode to the imaging ready state; however, controls, prior to the switching, the battery remaining power detecting section 40 to detect the remaining power of the battery 21. Moreover, when the radiographer directly operates the input operation unit 26 of the detector 5 to input the radiographing instruction, the controller 27 controls respective running states of the plurality of drive units based on the radiographing instruction to thereby control the power consumption of the battery 21 for switching from the first imaging standby mode to the imaging ready state; however, controls prior to the switching the battery remaining power detecting section 40 to detect the remaining power of the battery 21.

When the detected result of the remaining power obtained by the remaining power detecting section 40 satisfies the power possible to radiographto radiograph, the controller 27 controls respective running states of the plurality of drive units to thereby control the power consumption of the battery 21 so that the detector 5 can be switched to the imaging ready state. At this time, the controller 27 outputs to the console 6 through the communication unit 24 a message that the radiographing is ready. Based on the signal input to the communication unit 33, the console 6 controls the display unit 32 to indicate that the radiographing is permitted.

On the other hand, when the detected result of the remaining power obtained by the remaining power detecting section 40 is less than the power possible to radiographto radiograph, the controller 27 controls respective running states of the plurality of drive units to thereby control the power consumption of the battery 21 so that the detector 5 can go into the second imaging standby mode of the imaging standby state. At this time, the controller 27 controls the indicator 25 to indicate that the radiographing is not permitted, and outputs to the console 6 through the communication unit 24 a message that the radiographing is not permitted. Based on the signal input to the communication unit 33, the console 6 controls the display unit 32 to indicate that the radiographing is not permitted.

In this case, the controller 27 outputs to the console 6 through the communication unit 24 the detected result of the remaining power detecting section 40 and the state of the radiation image detector 5 (the imaging ready state or the imaging standby state). Based on the signal input to the communication unit 33, the console 6 controls the display unit 32 to display the remaining power of the battery 21 and the state of the radiation image detector 5.

As described above, according to the embodiment, when the radiographing instruction is input to the controller 27 of the radiation image detector 5 through the input operation unit 26 or the communication unit 24, the battery remaining power detecting section 40 detects the remaining power of the battery, which allows recognition of the remaining power of the battery prior to the radiographing. This allows determination as to whether normal radiographing is possible to be performed prior to the radiographing, resultantly allows prevention of radiographing under insufficient remaining power of the battery. The prevention of radiographing under insufficient remaining power of the battery suppresses frequency of re-radiographing, and prevents a patient from unnecessary exposure to radiation.

Moreover, in the case that the detected result of remaining power, in the battery remaining power detecting section 40 at the time when the radiographing instruction is input to the controller 27, satisfies the power possible to radiographto radiograph, the radiation image detector goes into the imaging ready state, and goes into the imaging standby state in the case that the detected result is less than the power possible to radiographto radiograph. Therefore, it is securely prevented to carry out radiographing with the remaining power less than the power possible to radiographto radiograph. Moreover, when the remaining power is less than the power possible to radiographto radiograph, the indicator 25 and the console 6 notify that the radiographing is not permitted, and therefore the radiographer can carry out, e.g., replacement, charging of the battery, and the like based on the notice.

The imaging standby state includes a plurality of modes with respective different power consumptions (the first and second imaging standby modes), and therefore the radiation image detector can be set to the most suitable state according to, e.g., its use condition. This allows suppression of useless power consumption, and allows efficient radiographing work.
Moreover, in the case that the detected result of remaining power, in the battery remaining power detecting section 40 at the time when the radiographing instruction is input to the controller 27, is less than the power possible to radiographto radiograph, the detector goes into the mode of minimum power consumption (the second imaging standby mode) out of the plurality of modes in the imaging standby state, and therefore the power consumption can be reduced as much as possible when radiographing is not permitted.

It is apparent that the invention is not limited to the above-described embodiment and can be modified as appropriate.
For example, two kinds of modes are selectable as an imaging standby state in this embodiment, but the imaging standby state is not limited to the two kinds described above. For example, there may be employed an imaging standby mode in which supplying of power is stopped only to photodiodes 233 and TFTs 234 which have a characteristic of deterioration with time when power is supplied, and another imaging standby mode in which, while supplying of power is stopped to all units except the image storing unit 18 and the communication unit 24, the power is supplied to photodiodes 233 and TFTs 234 prior to the other units because they need longer time for turning on again when the power supply is once stopped, and further, plural kinds of modes may be selected. Moreover, the detector may have only either of the two imaging standby modes described in this embodiment as examples.

There is described as an example in this embodiment such that the detection of remaining power of the battery 21 by the remaining power detecting section 40 is performed prior to switching from the first imaging standby state to the imaging ready state, but alternatively the detection of remaining power may be performed right after the switching. This "right after the switching" means a state that radiographing is not performed yet after switching to the imaging ready state.
In this embodiment, the power supply 19 is configured to have the chargeable battery 21 in addition to the auxiliary battery 20. However, the configuration of the power supply 19 is not limited thereto, and the power supply 19 may have a replaceable and disposable battery in addition to the auxiliary battery.

In order to charge the chargeable battery 21, a charging device, such as a cradle, is used in this embodiment, but by connecting the terminal of the radiation image detector having a cord for supplying power, an external power supply may supply power to charge the battery. Moreover, the battery may be charged while it is taken out of the radiation image detector.

As for the switching unit that gives an instruction (switching instruction) of switching between the imaging ready state and the imaging standby state, the communication unit 24 and the input operation unit 26 of the detector 5 are used as examples in this embodiment, but there can be used as the switching unit the console 6, a mechanical switch other than these units, an electric signal, a sensor, etc.
When the console 6 is used as the switching unit, there can be used as an instruction signal, for example, selection information of a patient, the information input after the radiation image detector 5 to be used for radiographing is selected, a power-ON signal, ON/OFF signals of other switches, etc.
When a signal from the detector 5 are used as a switching instruction, there can be used, for example, a signal from switches or sensors (acceleration sensor, contact sensor, etc), a signal generated when the detector contacts an external device such as a cradle, etc.
By using the server 2 or the radiation source 12 as a switching unit, a signal from these devices may be used as a switching instruction.

Such a case is explained as an example in this embodiment that detection of remaining power by the battery remaining power detecting section 40 is performed only when the radiographing instruction is input to the controller 27. However, it is also possible to detect the remaining power by the battery remaining power detecting section 40 when the detection of remaining power is instructed from the input operation unit 26 or the console 6. Moreover, it is also possible to detect the remaining power automatically at predetermined intervals when the detection of remaining power by the battery remaining power detecting section 40 has not been performed for a certain period. Moreover, when radiographing is performed continuously, the detector is always in the imaging state, and it is therefore preferable to detect the remaining power with the remaining power detecting section 40 every time of radiographing. In this case, the timing of detecting the remaining power may be before or after the radiographing.

In addition to the detection of remaining power of the battery 21 by the battery remaining power detecting section 40, various operation checks may be employed. There may be employed, for example, a reading operation check for checking whether an image can be read normally, a transfer-operation check for checking whether an image can be transferred normally, a wireless operation check for checking whether a signal can be communicated normally with the console 6 or the server 2, a memory check for checking whether the internal memory works normally, and so on. Determination data necessary for respective determinations are stored in the ROM of the control device 28 in the radiation image detector 5.

### [Second Embodiment]

In the first embodiment, such a case has been explained as an example that, when an radiographing instruction is input from the input operation unit 26 or the communication unit 24, the controller 27 controls the battery remaining power detecting section 40 to detect the remaining power of the chargeable battery 21. In a second embodiment, when an instruction for switching from the second imaging standby mode to the first imaging standby mode, that is, an instruction for switching from the imaging standby mode of less power consumption to the imaging standby mode of larger power consumption out of the plurality of imaging standby modes (a standby-state switching instruction) is input from the input operation unit 26 or the communication unit 24, the controller 27 also controls the battery remaining power detecting section 40 to detect the remaining power of the chargeable battery 21. In the second embodiment, elements (devices, units, or sections) which are the same as corresponding elements in the first embodiment are designated by the same reference numerals and the description thereof is omitted.

The photodiodes 233 and the TFTs 234 need a longer time to go into their stable states when power is supplied again after supplying of power is stopped. Therefore, when radiographing is not performed for a while, the second imaging standby mode, in which power is not supplied to the photodiodes 233 and the TFTs 234, is set, and when radiographing will soon be performed, the first imaging standby mode, in which power is supplied to the photodiodes 233 and the TFTs 234, is set. When the second imaging standby mode is transferred to the first imaging standby mode, it is probable that radiographing will soon be performed, and hence the remaining power of the battery 21 is detected when the standby-state switching instruction is input.

In this case, operation of the input operation unit 26 also allows switching to be set under the plurality of imaging standby states. For example, when the instruction of switching to the first imaging standby mode is input to the input operation unit 26, or when the instruction signal of switching to the first imaging standby mode is input to the communication unit 24 from the console 6, the detector goes into the first imaging standby mode. On the contrary, when the instruction of switching to the second imaging standby mode is input to the input operation unit 26, or when the instruction signal of switching to the second imaging standby mode is input to the communication unit 24 from the console 6, the detector goes into the second imaging standby mode. That is, the input operation unit 26 and the communication unit 24 act as a switching unit for giving an instruction of switching the plurality of imaging standby states according to the invention.

A description will now be given of an action of the radiation imaging system 1 including the radiation image detector 5 applied thereto according to this embodiment.

When a reservation of radiographing is not input to the radiation image detector 5, the controller 27 of the radiation image detector 5 normally controls respective running states of the plurality of drive units to be in the second imaging standby mode for reducing the power consumption in the standby state.

Thereafter, when an radiographing-reservation instruction is input to the console 6, a radiographer selects a radiation image detector 5 to be used for the radiographing on the console 6, and inputs the contents to the input operation unit 31 of the console 6. The input contents are transmitted to the communication unit 24 of the selected detector 5 through the communication unit 33 of the console 6, and are input to the controller 27 as the standby-state switching instruction information. Based on the standby-state switching instruction information, the controller 27 controls the power consumption of the chargeable battery 21 for switching from the second imaging standby mode to the first imaging standby mode; however, controls, prior to the switching, the battery remaining power detecting section 40 to detect the remaining power of the battery 21. Moreover, when the radiographer directly operates the input operation unit 26 of the detector 5 to input the standby-state switching instruction, the controller 27 controls respective running states of the plurality of drive units based on the standby-state switching instruction to thereby control the power consumption of the battery 21 for switching from the second imaging standby mode to the first imaging standby mode; however, controls, prior to the switching, the battery remaining power detecting section 40 to detect the remaining power of the battery 21.

When the detected result of the remaining power obtained by the remaining power detecting section 40 satisfies the power possible to radiographto radiograph, the controller 27 controls respective running states of the plurality of drive units to thereby control the power consumption of the battery 21 so that the detector 5 can be switched to the first imaging standby mode. At this time, the controller 27 outputs to the console 6 through the communication unit 24 a message that the radiographing is possible. Based on the signal input to the communication unit 33, the console 6 controls the display unit 32 to display the message that the radiographing is possible.

On the other hand, when the detected result of the remaining power obtained by the remaining power detecting section 40 is less than the power possible to radiographto radiograph, the controller 27 controls respective running states of the plurality of drive units to thereby control the power consumption of the battery 21 so that the detector 5 can go into the second imaging standby mode. At this time, the controller 27 controls the indicator 25 to indicate that the radiographing is not permitted, and outputs to the console 6 through the communication unit 24 a message that the radiographing is not permitted. Based on the signal input to the communication unit 33, the console 6 controls the display unit 32 to display the message that the radiographing is not permitted.

As described above, according to this embodiment, when the standby-state switching instruction is input to the controller 27 of the radiation image detector 5 through the input operation unit 26 or the communication unit 24, the battery remaining power detecting section 40 detects the remaining power of the battery, and therefore the remaining power of the battery can be recognized prior to the radiographing. This allows determination as to whether normal radiographing is possible to be performed prior to the radiographing, therefore allows prevention of radiographing under insufficient remaining power of the battery. The prevention of radiographing under insufficient remaining power of the battery suppresses frequency of re-radiographing, and prevents a patient from unnecessary exposure to radiation.

Moreover, in the case that the detected result of remaining power of the battery remaining power detecting section 40, at the time when the standby-switching instruction is input to the controller 27, satisfies the power possible to radiographto radiograph, the detector goes into the first imaging standby mode, and goes into the second imaging standby mode in the case that the detected result is less than the power possible to radiographto radiograph. Therefore, it is securely prevented to carry out radiographing with the remaining power less than the power possible to radiographto radiograph. Moreover, when the remaining power is less than the power possible to radiographto radiograph, the indicator 25 and the console 6 notify that the radiographing is not permitted, and therefore the radiographer can carry out, e.g., replacement, charging or the like of the battery based on the notice.

In the case that the detected result of remaining power of the battery remaining power detecting section 40, at the time when the standby-switching instruction is input to the controller 27, is less than the power possible to radiographto radiograph, the detector is in the state of minimum power consumption (the second imaging standby mode) out of the plurality of the imaging standby states, and therefore the power consumption can be reduced as much as possible when radiographing is not permitted.

It is apparent that the invention is not limited to the above-described embodiment and can be modified as appropriate.

As for the switching unit that gives a standby-state switching instruction, the communication unit 24 and the input operation unit 26 of the detector 5 are used as examples in this embodiment, but there can be used as the switching unit a mechanical switch other than these units, an electric signal, a sensor, etc.

Such a case has been explained as an example in this embodiment that, when the radiation image detector 5 to be used for radiographing is input to the input operation unit 31 of the console 6, the input contents are treated as standby-state switching instruction information, but the standby-state switching instruction information is not limited to this case, and other signals input to the console 6 may be treated as the standby-state switching instruction information. For example, there can be used selection information of a patient, the information input after the radiation image detector 5 to be used for radiographing is selected, a power-ON signal, ON/OFF signals of other switches, etc.
Other than these signals, a signal from the detector 5, the server 2 or the radiation source 12 may be used as the standby-state switching instruction information.
When a signal from the radiation image detector 5 is used as the standby-state switching instruction information, there can be used, for example, a signal from switches or sensors (acceleration sensor, contact sensor, etc), a signal generated when the detector contacts an external device such as a cradle.

### [Third Embodiment]

Such a case is explained in the first embodiment that the remaining power of the battery is detected by the battery remaining power detecting section 40, and in harmonization with this, in a third embodiment, a description will be given of a case that the check unit checks drive units as to whether the drive units can perform respective operations normally at the time when the detector starts working. In the third embodiment, elements (devices, units, or sections) which are the same as corresponding elements in the first embodiment are designated by the same reference numerals and the description thereof is omitted.

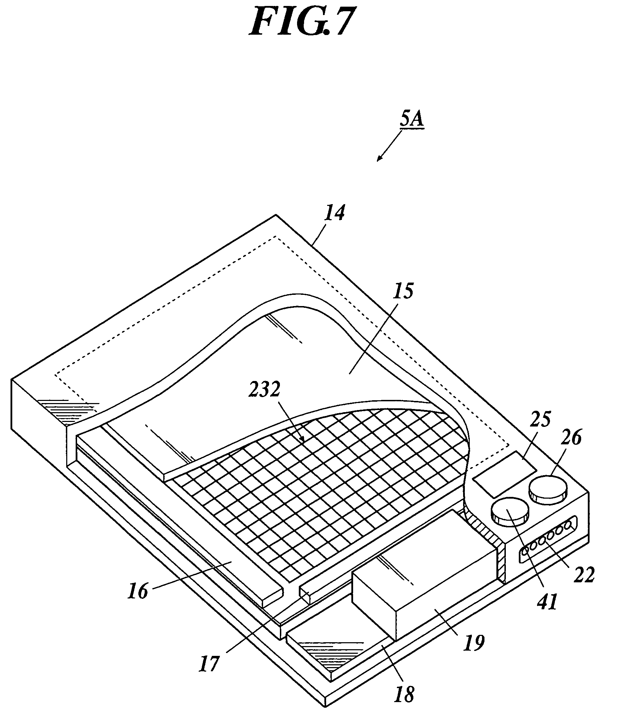

As shown in FIG. 7, on the casing 14 of a radiation image detector 5A according to the third embodiment, a start switch 41 is mounted for turning ON/OFF a main power source of the radiation image detector 5 and for inputting a start instruction and a start-halt instruction of the radiation image detector 5. By operating the start switch 41 and the input operation unit 26, the operation state of the radiation image detector 5 can be set for switching. The start switch 41 is used, for example, when the battery of the detector 5 is replaced, and thus used by very few frequencies. Therefore, it is preferable to mount the start switch inside a door at a position difficult to be touched such that the door is, for example, provided so as to be opened or closed at a part of the casing 14 and the switch is operable with the door opened. Such arrangement of the start switch 41 prevents the operator from erroneously touching the switch and causing occurrence of malfunction of the radiation image detector 5.
In the third embodiment, the radiation image detector 5A is shown as an example such that the power supply 19 includes only a chargeable battery.

The operation state of the radiation image detector 5 will now be explained.
The operation state of the radiation image detector 5 includes an OFF state and ON state of the main power source. In the OFF state of the main power source, power is completely turned off in all drive units of the detector 5, and the power supply from the chargeable battery is completely shut off. On the contrary, In the ON state of the main power source, the power from the battery is supplied to respective drive units of the detector 5, and the detector includes the imaging ready state in which radiographing operation can be performed and the imaging standby state in which power consumption is less than that of the imaging ready state.

As for switching of the aforementioned operation states of the radiation image detector 5, the detector is configured to be switched to the ON state of the main power source when the start instruction is input with operation of the start switch 41, and switched to the OFF state of the main power source when the start-halt instruction is input. The switching of respective operation states included in the ON state of the main power source is performed based on the instruction input to the input operation unit 26 or the communication unit 24, that is, when the radiographing instruction or standby instruction is input with operation of the input operation unit 26, or when the radiographing instruction signal or standby instruction signal is input to the communication unit 24.

Specifically, when the start instruction is input with operation of the start switch 41 in the OFF state of the main power source, the detector is configured to be switched to a predetermined imaging standby state from the OFF state of the main power source. When the radiographing instruction is input to the input operation unit 26 or the radiographing instruction signal is input to the communication unit 24 from the console 6 in the first imaging standby mode of the imaging standby state, the detector is configured to be switched to the imaging ready state from the first imaging standby mode. When the standby instruction is input to the input operation unit 26 or the standby instruction signal is input to the communication unit 24 from the console 6 in the first imaging standby mode, the detector may be configured to be switched to the second imaging standby mode from the first imaging standby mode.

Thus, switching of operation states in the radiation image detector 5 is performed based on the instruction from the start switch 41, the input operation unit 26 or the communication unit 24. In the operation states according to the invention, the start switch 41 is the switching unit for giving the instruction of switching between the ON state and OFF state of the main power source, and the input operation unit 26 or the communication unit 24 is the switching unit for giving the instruction of switching between the imaging ready state and the imaging standby state. Alternatively, the switching from the ON state to the OFF state of the main power source may be also performed based on the instruction from the communication unit 24.

The radiation image detector 5 has a check unit for checking whether operations of respective drive units can be performed normally. The operation check in this embodiment includes power check for the power supply 19, communication check for the communication unit 24 and memory check for the image storing unit 18, and the check unit include respective check operations. Each check unit will be explained below.

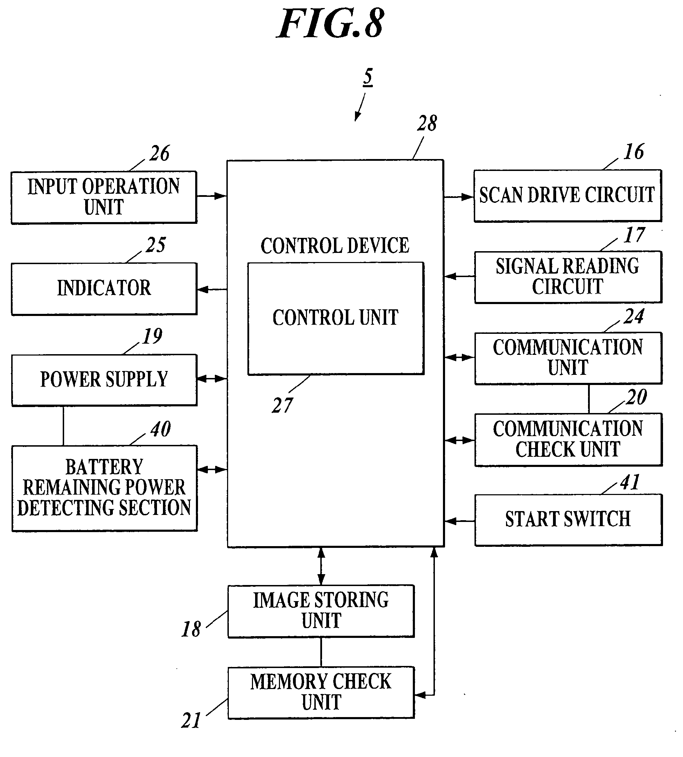

A check unit for the power check may correspond to the battery remaining power detecting section 40 shown in FIG. 8. The remaining power detecting section 40 detects the remaining power of the battery as the remaining power of the power supply 19 according to control of the controller 27, checks whether the obtained remaining power is not less than the predetermined power possible to radiographto radiograph, and outputs the obtained result to the controller 27.

There is provided with a communication check unit 20 as a check unit for the communication check. The communication check unit 20 checks according to control of the controller 27 whether the detector can transmit and receive signals to and from the console 6 or the server 2 normally, or can transmit an image normally, and outputs the obtained result to the controller 27.

There is provided with a memory check unit 21 as a check unit for the memory check. The memory check unit 21 checks according to control of the controller 27 whether the internal memory can work normally, and outputs the obtained result to the controller 27.

As for timing of the operation check by these check units, various timings are possible to be employed, but in this embodiment, the check is performed at the time of starting operation. When the start switch 41 inputs the instruction of switching from the OFF state to the ON state of the main power source (start instruction) to the controller 27, the controller 27 controls the operation check unit to perform respective operation checks.

When the start instruction is input by the start switch 41, the controller 27 detects respective check unit, and switches between the OFF state and the ON state of the main power source based on the result of operation checks of respective check units. At this time, when each drive unit for the check to be performed is once operated, the check unit performs operation check in this state, and after the operation check, the drive unit is switched to a predetermined operation state. When the check unit detects that the drive unit for the check to be performed cannot work normally, the controller 27 does not permit the detector at least to be switched to the imaging ready state. Particularly, when detected that the power supply 19 cannot work normally, the detector goes into the OFF state of the main power source, and when detected that the communication unit 24 and the image storing unit 18 cannot work normally, the detector is preferably configured to go into the second imaging standby mode. Here, the case of detection that the power supply 19 cannot work normally means that the battery remaining power detecting section 40 detects that the remaining power of the battery is less than the predetermined power possible to radiographto radiograph.

Specifically, when the start instruction is input, the controller 27 compares the results of power check, communication check and memory check with respective determination data stored in the ROM. When the remaining power of the battery is not less than the predetermined power possible to radiographto radiograph and the communication unit 24 and the image storing unit 18 are detected to work normally, the controller 27 enables the power supply to start supplying power from the battery so that the detector can go into the first imaging standby mode capable of radiographing immediately out of the imaging standby state, and controls the power supplied to respective drive units to thereby control the running state of the plurality of drive units. When the remaining power of the battery is not less than the predetermined power possible to radiograph and the communication unit 24 or the image storing unit 18 is detected not to work normally, the controller 27 enables the power supply to start supplying power from the battery so that the detector can go into the imaging standby mode of minimum power consumption out of the imaging standby state, namely, the second imaging standby mode, and controls the power supplied to respective drive units to thereby control the running state of the plurality of drive units. When the controller 27 detects that the remaining power of the battery is less than the predetermined power possible to radiograph, the controller shuts off the power supply to respective drive units, the power supplied from the battery at the time of operation checks, to thereby control the running state of the plurality of drive units. Accordingly, by controlling the running state of the plurality of drive units, overall power consumption of the radiation image detector 5 is controlled.

Moreover, the controller 27 enables the indicator 25 to display the result of operation checks performed by respective check units. Specifically, when remaining power of the battery is not less than the power possible to radiograph and the results of communication check and memory check are normal, the controller 27 controls the indicator 25 to display that the radiographing is permitted. When remaining power of the battery is not less than the power possible to radiograph and the result of either communication check or memory check is not normal, the controller 27 controls the indicator 25 to display that operation is unable to be performed normally. Further, when the result of communication check is normal, the controller 27 transmits the above display signals, as information about operation states of respective drive units, to the console 6 through the communication unit 24.

A description will now be given of an action of the radiation imaging system 1 including the radiation image detector 5A according to this embodiment applied thereto.

Usually, when the radiation image detector 5A is in the OFF state of the main power source, power is completely shut off in all drive units of the radiation image detector 5A.

When the radiographer operates the start switch and the radiation image detector 5A is switched to the ON state of the main power source, the controller 27 controls the running states of respective drive units to switch from the OFF state of the main power source to a predetermined imaging standby state. Prior to the switching, the remaining power detecting section 40, the communication check unit 20 and the memory check unit 21 are controlled to perform detection of remaining power of the battery, communication check of the communication unit 24, and memory check of the image storing unit 18.

When the controller 27 detects that the remaining power of the battery is not less than the predetermined power possible to radiograph and the communication unit 24 and the image storing unit 18 work normally, the controller 27 controls the power supplied to respective drive units from the battery so that the detector can be switched to the first imaging standby mode, to thereby control the running states of respective drive units. At this time, the controller 27 controls the indicator 25 to display a message that the radiographing is possible, and transmits to the console 6 through the communication unit 24 the message that the radiographing is possible, then based on the signal input to the communication unit 33, the console 6 controls the display unit 32 to display the message that the radiographing is possible.

On the other hand, when the controller 27 detects that the remaining power of the battery is not less than the predetermined power possible to radiograph and the communication unit 24 or the image storing unit 18 cannot work normally, the controller 27 controls the power supplied to respective drive units from the battery so that the detector can be switched to the second imaging standby mode, to thereby control the running states of respective drive units. At this time, the controller 27 controls the indicator 25 to display a message that one of the communication unit 24 and the image storing unit 18, which is detected unable to work normally, cannot work normally. When the controller 27 detects that the remaining power of the battery is not less than the predetermined power possible to radiograph and the image storing unit 18 cannot work normally, the controller 27 transmits to the console 6 through the communication unit 24 the message that the image storing unit 18 cannot work normally. Based on the signal input to the communication unit 33, the console 6 controls the display unit 32 to display the message that the image storing unit 18 cannot work normally. Watching the indicator 25 or the display unit 32, the radiographer, e.g., repairs the drive unit displayed unable to work normally to solve the malfunction, thereafter uses the detector again for radiographing.

When the controller 27 detects that the remaining power of the battery is less than the predetermined power possible to radiograph, the controller shuts off the power supply from the battery to respective drive units so that the detector goes into the OFF state of the main power source, to thereby control respective drive units. Watching that the radiation image detector 5A does not start operating, the radiographer, e.g., mounts the radiation image detector 5 onto the charging device 23 for charging or replaces the battery to solve the malfunction of the power supply 19, thereafter uses the detector again for radiographing.

Thereafter, the controller 27 of the detector 5A, which is switched into the first imaging standby mode, detects through the input operation unit 26 or the communication unit 24 whether either the radiographing instruction information or the standby instruction information is input.

At this time, when radiographing-reservation instruction information is input to the console 6, the radiographer selects a radiation image detector 5A to be used for the radiographing on the console 6, and inputs the contents to the input operation unit 31 of the console 6. The input contents are transmitted to the communication unit 24 of the selected detector 5A through the communication unit 33 of the console 6, and are input to the controller 27 as the radiographing instruction information. Alternatively, when the radiographer directly operates the input operation unit 26 of the detector 5A to be used for radiographing after the input of the radiographing-reservation instruction, the radiographing instruction is also input as the radiographing instruction information. Based on the radiographing instruction information, the controller 27 controls respective running states of the plurality of drive units to thereby switch from the first imaging standby mode to the imaging ready state, and then imaging operation is performed.

On the other hand, if the radiographer selects a radiation image detector 5A to be switched to the second standby mode on the console 6 and inputs the contents to the input operation unit 31 of the console 6 before the radiographing-reservation instruction information is input to the console 6, then the input contents are transmitted to the communication unit 24 of the selected detector 5A through the communication unit 33 of the console 6, and are input to the controller 27 as the imaging standby instruction information. Alternatively, when the radiographer directly operates the input operation unit 26 of the detector 5A to be switched to the second standby mode before the radiographing-reservation instruction is input to the console 6, the standby instruction is also input as the standby instruction information. The controller 27 controls respective running states of the plurality of drive units based on the standby instruction information, to thereby switch from the first radiographing standby mode to the second radiographing standby mode.

As described above, according to this embodiment, when the start instruction is input to the controller 27 of the radiation image detector 5A through the start switch 41, the check unit for the battery, communication unit 24 and image storing unit 18 check whether the units can operate normally when the detector starts operating. Therefore, it is determined prior to radiographing whether the radiographing can be performed normally. This prevents the radiographing from being performed with units malfunctioning. This can therefore suppress the frequency of re-radiographing due to malfunction of units, and can prevent a patient from unnecessary exposure to radiation.

The controller 27 controls operation states of respective drive units according to the result of operation checks, and when determined that any drive unit cannot work normally according to the result of operation checks, the controller 27 does not switch the radiation image detector 5A at least to the imaging ready state. Particularly, when the remaining power of the battery is less than the predetermined power possible to radiograph, the controller causes the radiation image detector 5A to go into the OFF state of the main power source, and it is therefore securely prevented that radiographing is performed with the battery malfunctioning.

Moreover, the indicator 25 and the console 6 notify that the radiographing is permitted when the remaining power of the battery, at the time when the start instruction is input to the controller 27, is not less than the predetermined power possible to radiograph and the results of operation check of the communication check unit 20 and the memory check unit 21 are respectively normal, and notify that the detector cannot work normally when the remaining power of the battery is not less than the predetermined power possible to radiograph and the results of operation check for the communication check unit 20 and the memory check unit 21 are not normal. Based on the notification, the radiographer can deal with the corresponding malfunction of drive units.

Moreover, the imaging standby state includes a plurality of modes with respective different power consumptions (the first and second imaging standby modes), and therefore the radiation image detector can be set to the most suitable state according to, e.g., its use condition. This allows suppression of useless power consumption, and allows efficient radiographing work.

Particularly, when the remaining power of the battery, at the time when the start instruction is input to the controller 27, is not less than the predetermined power and the result of operation checks of other units is not normal, the detector is in the mode of minimum power consumption (the second imaging standby mode) out of the plurality of modes in the imaging standby state, and therefore the power consumption can be reduced as much as possible when the battery has little remaining power.

It is preferable that the radiation image detector 5A is used as a cordless system, because the cordless system improves flexibility of radiographing operation and resultantly improves overall operability compared with the case that the detector is used as a wired system having a cord or the like connected thereto. Particularly, a wireless communication system allows speedy transmission/reception of information such as images at the time of communication. At this time, the cordless radiation image detector cannot always perform the communication check and the charging check because the detector does not always communicate with the console, being different from the wired system. Accordingly, application of the present invention allows achieving a greater effect.

It is apparent that the invention is not limited to the above-described third embodiment and can be modified as appropriate.
For example, there are provided as check units with the battery remaining power detecting section 40, the communication check unit 20 and the memory check unit 21 for performing respective operation checks of drive units in this embodiment, but various operation checks may be performed in other drive units. In this case, one check unit may be configured to perform a plurality of operation checks.

As a specific operation check, there may be employed a read operation check of the signal reading circuit 17 for checking whether an image can be read normally. There may be also an operation check for checking whether photodiodes 233 and TFTs 234 function normally.

In this case, the radiation image detector 5A may be controlled not to be switched at least to the imaging ready state when the controller 27 determines that these drive units cannot work normally. When any abnormality is found in the radiation image detector 5A, the detector may be restarted. Incidentally, determination data necessary for determining respective operation checks may be stored in the ROM of the control device 28 in the radiation image detector 5A.

As for the switching unit that gives an instruction of switching operation states during the ON state of the main power source in the radiation image detector 5A, the communication unit 24 and the input operation unit 26 of the detector 5A are used as examples in this embodiment, but a sensor arranged in the radiation image detector 5A can be used as the switching unit. The sensor may include, e.g., an acceleration sensor and a contact sensor. With these sensors, the operation states during the ON state of the main power source in the detector 5A may be switched by detecting the change of acceleration and a pressure of the detector 5A given when the radiation image detector 5A gets in contact or noncontact with an external device such as a cradle.

The operation states during the ON state of the main power source in the radiation image detector 5A is switched by selecting and inputting a radiation image detector 5A to be used for radiographing to the input operation unit 31 of the console 6. At this time, a patient to be imaged may be selected simultaneously. Alternatively, turning on the console 6 may switch the operation states during the ON state of the main power source for the radiation image detector 5A that has been registered in the console 6 in advance.

Moreover, input to the controller 27 is not limited to the input from the radiation image detector 5A or the console 6, and may be that from other external devices provided on the network 7, such as a host computer controlling the console 6, and the radiation source 12.

In this embodiment, when the start instruction is input to the controller 27, that is, after starting, operation checks by the battery remaining power detecting section 40, the communication check unit 20 and the memory check unit 21 have been performed before the detector is switched to a predetermined operation state, but these operation checks may be appropriately performed during the start operation after the detector is once switched to the predetermined operation state. In this case, as the timing of performing operation checks, for example, operation checks by the check unit can be performed when the operation check is instructed from the input operation unit 26 or the console 6. Furthermore, when the operation checks are not performed by the check unit for a certain period during the start operation after the detector is switched to the predetermined operation state, the operation checks may be performed automatically at predetermined intervals. In case of continuous radiographing, the detector is always in an imaging state, and therefore the operation checks may be performed by the check unit preferably every time radiographing is carried out. In this case, the operation checks may be performed before or after the radiographing.

In this embodiment, a radiation image detected at the radiation image detector 5A is sent to the console 6 and image processing is executed, but the destination for the radiation image to be sent to and the place of executing image processing may be other external devices such as a host computer and the server 2.

Moreover, in this embodiment, the display unit 32 of the console 6 is enabled to display the result of operation checks and also function as a notifying unit, but the display unit 32 may be further enabled to display the running state of the radiation image detector 5.

### Explanation of Reference Numeral

- 1: radiation imaging system
- 2: server
- 3: radiographing operation device
- 4: base station
- 5: radiation image detector
- 6: console
- 7: network
- 10: radiation imaging device
- 16: scan drive circuit
- 17: signal reading circuit
- 18: image storing unit
- 19: power supply
- 20: auxiliary battery
- 21: chargeable battery (battery)
- 23: charging device
- 24: communication unit
- 26: input operation unit
- 27: control unit
- 40: battery remaining power detecting section

## Claims

1. A radiation image detector switchable between an imaging ready state which can detect radiation and an imaging standby state in which power consumption is less than that of the imaging ready state, the detector comprising:
a switching unit for giving an instruction of switching between the imaging ready state and the imaging standby state;
a chargeable or replaceable battery provided as a power supply source for supplying power to a plurality of drive units;
a battery remaining power detecting section for detecting a remaining power of the battery; and
a control unit for controlling running states of the plurality of drive units to switch between the imaging ready state and the imaging standby state according to the instruction from the switching unit and for controlling the battery remaining power detecting section,
wherein the control unit controls the running states of the plurality of drive units to switch from the imaging standby state to the imaging ready state on the basis of a detected result of the remaining power of the battery, by the battery remaining power detecting section.

2. The radiation image detector of claim 1, wherein the control unit controls the running states of the plurality of drive units to switch between the imaging ready state and the imaging standby state on the basis of the detected result of the remaining power by the battery remaining power detecting section at the time when an imaging instruction for switching from the imaging standby state to the imaging ready state is input from the switching unit.

3. The radiation image detector of claim 2, wherein the control unit controls the running states of the plurality of drive units such that the detector goes into the imaging ready state when the detected result of the remaining power in the battery remaining power detecting section, at the time when the imaging instruction is input from the switching unit, satisfies the power possible to radiograph, and goes into the imaging standby state when the detected result is less than the power possible to radiograph.

4. The radiation image detector of claim 3, further comprising a notifying unit for notifying on the basis of the control of the control unit, wherein the control unit controls the notifying unit to notify that the imaging is not permitted when the detected result of the remaining power in the battery remaining power detecting section, at the time when the imaging instruction is input from the switching unit, is less than the power possible to radiograph.

5. The radiation image detector of any one of claims 1 to 4, wherein the imaging standby state has a plurality of modes, and wherein the control unit controls the running states of the plurality of drive units so that the plurality of modes have respective different power consumptions.

6. The radiation image detector of claim 5, wherein the control unit controls the running states of the plurality of drive units such that the detector goes into a mode of minimum power consumption in the plurality of modes in the imaging standby state when the detected result of the remaining power in the battery remaining power detecting section, at the time when the imaging instruction is input from the switching unit, is less than the power possible to radiograph.

7. The radiation image detector of claims 5 or 6, wherein the control unit controls the battery remaining power detecting section to detect the remaining power of the battery when a standby-state switching instruction for switching from a mode of smaller power consumption to a mode of larger power consumption in the plurality of modes is input from the switching unit, the standby-state switching instruction.

8. The radiation image detector of any one of claims 1 to 7, further comprising:
a check unit for performing an operation check of the drive units as to whether the drive units can work normally when the detector starts to operate; and
a control unit for controlling the running states of the plurality of drive units on the basis of the result of the operation checks.

9. The radiation image detector of claim 8,
wherein the running state includes an ON state of a main power source, the ON state including an imaging ready state which can detect radiation and an imaging standby state in which power consumption is less than that of the imaging ready state; and an OFF state of the main power source in which power supply to the drive units is completely shut off, and
wherein when the check unit detects that one of the drive units cannot work normally, the detector is not switched at least to the imaging ready state.

10. The radiation image detector of claim 9, wherein when the battery remaining power detecting section detects that the remaining power of the power source is less than a predetermined power possible to radiograph, the control unit causes the detector to go into the OFF state of the main power source.

11. The radiation image detector of claim 9,
wherein the imaging standby state includes a first imaging standby mode, and a second imaging standby mode in which power consumption is less than that of the first imaging standby mode, and
the detector further comprises a communication unit as the drive unit; and a communication check unit for performing communication check of the communication unit as the operation check.

12. The radiation image detector of claim 11, wherein when the communication check unit detects that the communication unit cannot work normally, the control unit causes the detector to go into the second imaging standby mode.

13. The radiation image detector of claim 14, further comprising a notifying unit for performing notification on the basis of the control of the control unit, wherein when the communication check unit detects that the communication unit cannot work normally, the notifying unit notifies that the communication unit is impossible to work normally.

14. The radiation image detector of claim 9,
wherein the imaging standby state includes a first imaging standby mode, and a second imaging standby mode in which power consumption is less than that of the first imaging standby mode, and
the detector further comprises an image storing unit as the drive unit; and a memory check unit for performing a memory check of the image storing unit as the operation check.

15. The radiation image detector of claim 14, wherein when the memory check unit detects that the image storing unit cannot work normally, the control unit causes the detector to go into the second imaging standby mode.

16. The radiation image detector of any one of claims 1 to 15, wherein the detector is a cassette-shaped flat panel detector which detects irradiated radiation, converts the radiation to electric signals, accumulates the electric signals, and reads the accumulated electric signals to obtain radiation image data.

17. A radiation imaging system comprising:
the radiation image detector of any one of claims 1 to 16; and
a console which controls the radiation image detector.

18. The radiation imaging system of claim 17,
wherein the console comprises a display unit which displays on the basis of the control of the control unit, and
the control unit controls the display unit to display that the imaging is not permitted when the detected result of the remaining power in the battery remaining power detecting section, at the time when the imaging instruction is input from the switching unit, is less than the power possible to radiograph.

19. The radiation imaging system of claim 18, wherein the control unit controls the display unit to display the remaining power of the battery on the basis of the detected result in the battery remaining power detecting section.

20. The radiation imaging system of claims 18 or 19, wherein the control unit controls the display unit to display whether the radiation image detector is in the imaging ready state or the imaging standby state.

21. The radiation imaging system comprising:
the radiation image detector of any one of claims 8 to 15; and
a console which controls the radiation image detector,
wherein the console comprises a notifying unit which notifies either one or both of the running state of the radiation image detector and the result of operation checks of the radiation image detector.
